# EUROPÄISCHE PATENTSCHRIFT

(11) **EP 3 897 774 B1**
(45) Veröffentlichungstag und Bekanntmachung des Hinweises auf die Patenterteilung: **06.11.2024**
(21) Anmeldenummer: 19835374.0
(22) Anmeldetag: 18.12.2019
(51) Int. Cl.: A61M 1/36, A61M 39/02

(54) **VASKULÄRES ZUGANGSIMPLANTAT UND ZUGANGSIMPLANTATSYSTEM**
VASCULAR ACCESS IMPLANT AND ACCESS IMPLANT SYSTEM
IMPLANT D'ACCÈS VASCULAIRE ET SYSTÈME D'IMPLANT D'ACCÈS

(30) Priorität: 21.12.2018 DE 102018133404
(43) Veröffentlichungstag der Anmeldung: 27.10.2021
(73) Patentinhaber: B. Braun Avitum AG, 34212 Melsungen (DE)
(72) Erfinder: KRAUSE, Silvie, 34212 Melsungen (DE); RITTER, Kai-Uwe, 34212 Melsungen (DE); WEISE, Ben, 99735 Kleinwechsungen (DE); WOLFF, Henrik, 34212 Melsungen-Adelshausen (DE)
(74) Vertreter: Winter, Brandl - Partnerschaft mbB
(86) Internationale Anmeldenummer: PCT/EP2019/085855
(87) Internationale Veröffentlichungsnummer: WO 2020/127426

(56) Entgegenhaltungen:
- WO-A2-03/000314
- US-A1- 2014 276 345
- US-B2- 8 652 084

## Beschreibung

### Technisches Gebiet

Die vorliegende Erfindung betrifft ein vaskuläres Zugangsimplantat für einen Anschluss / eine Ankopplung / einen Zugang / eine Verbindung eines intrakorporalen Blutkreislaufs mit einem extrakorporalen Blutkreislauf, insbesondere zur (Fluid-) Verbindung mit einer extrakorporalen Blutbehandlungsmaschine wie einer Dialysemaschine, mit einer arteriellen Fluidleitung/Blutleitung, die dafür angepasst ist mit einer Arterie eines Patienten permanent (fluid-)verbunden zu sein, um der Arterie Blut zu entnehmen, mit einer venösen Fluidleitung/Blutleitung, die dafür angepasst ist, mit einer Vene des Patienten permanent (fluid-)verbunden zu sein, um der Vene Blut zuzuführen, sowie mit einer steuerbaren Arterie-Venen-Verbindung zwischen der arteriellen Fluidleitung und der venösen Fluidleitung, welche, insbesondere mittels eines steuerbaren Ventils, einen Fluidstrom zwischen der arteriellen Fluidleitung und der venösen Fluidleitung steuert. Daneben betrifft die Erfindung ein vaskuläres Zugangsimplantatsystem gemäß dem Oberbegriff des nebengeordneten Anspruchs.

### Stand der Technik

Für eine Verbindung eines intrakorporalen Blutkreislaufs mit einem extrakorporalen Blutkreislauf wird beispielsweise eine arteriovenöse Fistel als abnormale Verbindung zwischen einer Arterie und einer Vene eines Patienten geschaffen. Eine solche Verbindung, die auch Shunt genannt wird, ermöglicht einen höheren Blutfluss durch den extrakorporalen Blutkreislauf während einer Blutbehandlung.

Hierbei wird Blut aus der Arterie kommend in den extrakorporalen Blutkreislauf bzw. in einen ersten Anbindungsschlauch des extrakorporalen Blutkreislaufs abgeleitet, hiernach einer Behandlung unterzogen und anschließend insbesondere von einer Dialysemaschine kommend, der Vene des Patienten wieder zugeführt. Ein adäquater Blutfluss im extrakorporalen Kreislauf ist dabei eine wesentliche Bedingung für die optimale Entfernung der im Blut enthaltenen Toxine. Je nach extrakorporalen Verfahren werden sehr unterschiedliche Flüsse benötigt. Sie reichen von ca. 100 ml/min bei der Behandlung von Patienten auf einer Intensivstation bis zu 6000 ml/min bei Patienten an einer Herz-Lungen-Maschine. Bei einer Hämodialyse beispielsweise reicht in der Regel ein Anschluss bis maximal 400 ml/min aus.

Patienten mit chronischem Nierenversagen bekommen in der Regel eine am Arm chirurgisch geschaffene, arteriovenöse Fistel. Diese arteriovenöse Fistel dient bei einer Blutbehandlung als Gefäßzugang. Eine Vene hat sehr dünne Wände und einen zu geringen Blutdurchfluss. Die Venen können aber einen Durchmesser bekommen der für häufige Punktionen geeignet ist. Die Arterien hingegen haben ein zu geringes Volumen und liegen für Punktionen sehr ungeeignet, haben dafür aber den adäquaten Blutfluss. Ein Shunt ist eine arterialisierte Vene und aufgrund der Zunahme der Wanddicke für die häufigen (Venen) Punktionen ein geeignetes Blutgefäß, mit dem der Patient mehrmals pro Woche behandelt und mit Dialysekanülen punktiert werden kann.

Alternativ kann ein Mehrlumenkatheter in die obere oder untere Hohlvene platziert werden. Falls mehrere Lumina vorhanden sind, liegen diese in einem alle Lumina umfassenden Katheter. Da es nur einen Gefäßzugang gibt, müssen Blutentzug und Blutrückgabe intermittierend, also abwechselnd erfolgen. Dafür ist eine spezielle apparative Ausstattung des Dialysegeräts nötig. Beim Ansaugen des Blutes muss der venöse Blutschlauch insbesondere automatisch abgedrückt und somit das Blut am Zurückströmen gehindert werden. Ein spezieller Steuermechanismus regelt dabei die Einlauf-und Rücklaufphasen.

Aus der WO 2009/033177 A1 ist beispielsweise ein System bekannt, welches als Kathetersystem für eine Dialyse verwendet werden kann. Ein Implantat wird dabei unter die Haut des Patienten im Körper implantiert und mit einem Blutgefäß, wie einer Vene, verbunden. Das Implantat weist eine einzelne verschließbare Öffnung auf, durch welche ein Katheter hineingeführt und herausgenommen werden kann. Ein arterieller Fluss des Blutes wird dabei mittels eines Saug- bzw. Unterdrucks durch diese Öffnung angesaugt, an einen extrakorporalen Kreislauf abgegeben, behandelt, und hiernach durch einen flexiblen Schlauch, welcher ebenfalls durch die Öffnung in das Implantat hineinsteht und von dem angesaugten arteriellen Blut umgeben ist, dem intrakorporalen Blutkreislauf wieder zugeführt. Der flexible Schlauch weist eine lange Abmessung auf, um weit in die Vene hineinzustehen. Somit wird an einem einzigen Blutgefäß stromaufwärts das arterielle Blut abgeleitet, behandelt und stromab des Blutgefäßes wieder hinzugeführt.

Die WO 03/000314 A2 offenbart eine Fistel zwischen einer Arterie und einer Vene, eines Patienten, die mittels eines steuerbaren Ventils regelbar und geöffnet (Offen-Zustand) oder geschlossen (Geschlossen-Zustand) werden kann. Eine Steuereinheit regelt dabei das mechanische Ventil. Zwischenstufen zwischen dem Offen-Zustand und dem Geschlossen-Zustand sind nicht vorgesehen.

Die US 2004/0133173 A1 offenbart einen implantierbaren Zugang zur Anbindung des intrakorporalen Blutkreislaufs an einen extrakorporalen Blutkreislauf, hier für eine Hämodialyse, mit einer starren Struktur, die sowohl einen arteriellen Zugang als auch einen venösen Zugang aufweist. Sowohl der arterielle Zugang als auch der venöse Zugang weist eine Öffnung in einem Gehäuse auf, welche mechanisch verschlossen werden kann.

Die US 6,58 2,409 B1 offenbart eine implantierbare arteriovenöse Fistel welche eine Anbindung an eine Arterie sowie an eine Vene aufweist und für eine Hämodialyse geeignet ist. Diese implantierbare arteriovenöse Fistel weist einen speziell definierten Nadel Eintrittsbereich innerhalb eines Rahmens auf, der komplett im Körper aufgenommen ist. Über spezielle Nadeln, welche durch die Haut des Patienten hindurch gestochen werden und weiter in diesem Nadel Eintrittsbereich eingestochen werden, kann von der Arterie Blut entnommen werden und der Vene wieder zugeführt werden. Somit wird eine Verbindung zu einem extrakorporalen Blutkreislauf aufgebaut.

Die EP 1 622 657 B1 offenbart ein Dialyseventil mit einer Röhre, welche zwischen der Vene und der Arterie fluidverbunden ist und ferner einen pneumatisch betätigbaren Balg aufweist. Der Balg definiert eine innere Kammer, wobei die Röhre in der Kammer angeordnet ist, sodass, wenn der Balg sich in seiner Länge ändert, korrespondierend ein Abschnitt der Röhre sich ebenfalls im Durchmesser ändert. Hierdurch kann ein Fluss zwischen der Arterie und der Vene entsprechend des einstellbaren Durchmessers der Röhre gesteuert werden, und die Röhre funktioniert als einstellbare Drossel.

Die US 8,652,084 B2 offenbart ein vaskuläres Zugangsimplantat das einen arteriovenösen Shunt sowie entsprechende anastomotische Ventile aufweist. Speziell weist das implantierbare vaskuläre Zugangsimplantat einen ersten flexiblen Shunt auf, welcher Blut von dem arteriellen anastomotischen Ventil ausgehend zu einer Fluidsteuereinheit transportiert, sowie einen zweiten flexiblen auf, welcher das Blut von der Fluidsteuereinheit wieder zu dem venösen anastomotischen Ventil 14 transportiert. Für eine Anbindung eines externen Schlauchs wird eine Nadel durch die Haut des Patienten hindurch in den ersten flexiblen Shunt eingestochen.

Die US 2014/0276345 A1 offenbart ein vaskuläres Zugangsimplantat mit zwei Ventilen, welche miteinander über einen Schlauch verbunden sind. Die beiden Ventile können zwischen einer ersten und zweiten Position gedreht werden, um auf diese Weise einen Fluidfluss zu steuern.

All diese Systeme nach dem Stand der Technik weisen jedoch den Nachteil auf, dass der Patient mittels Nadeln gestochen werden muss, um einen Zugang zu dem Blutkreislauf zu erhalten, was Schmerzen für den Patienten bedeutet und die Organe schwächt und Blutgefäße schädigt, oder dass an nur einem Blutgefäß Blut entnommen und wieder abgegeben wird, was sich negativ auf das Herz auswirkt. Es bestehen ebenfalls Nachteile hinsichtlich einer Infektionsgefahr. Auch entsteht die Gefahr einer Erweiterung der Blutgefäße durch Erschlaffung der Gefäßmuskulatur, was als Vasodilatation bezeichnet wird. Aufgrund des größeren Widerstandes und einer einhergehenden erhöhten Leistungsbeanspruchung des Herzens tragen die üblichen Systeme zu einer Herzkrankheit als Konsequenz einer Vasodilatation bei. Auch besteht stets die Gefahr, dass sich die Nadeln lösen und es zu einem ungewollten Blutverlust an die Umgebung kommt.

### Zusammenfassung der Erfindung

Es ist daher die Aufgabe der Erfindung, die Nachteile aus dem Stand der Technik zu vermeiden oder wenigstens zu mindern und insbesondere ein vaskuläres Zugangsimplantat zur Verfügung zu stellen, das eine schmerzlose und sichere Verbindung/Anbindung eines intrakorporalen Blutkreislaufs an einen extrakorporalen Blutkreislauf erlaubt, die Sicherheit und Sterilität der Verbindung erhöht und einhergehend ein Infektionsrisiko senkt, autark funktionsfähig ist sowie körperlich nachteilige Nebeneffekte durch die dialyseseitige Behandlung vermeidet.

Die Aufgabe wird hinsichtlich des vaskulären Zugangsimplantats erfindungsgemäß durch die Merkmale des Anspruchs 1 und hinsichtlich des vaskulären Zugangsimplantatsystems durch die Merkmale des Anspruchs 12 gelöst.

Erfindungsgemäß ist das vaskuläre Zugangsimplantat also dafür angepasst, über einen mechanisch ausgebildeten arteriellen (Koppel-)Anschluss und einen mechanisch ausgebildeten venösen (Koppel-)Anschluss, lösbar, schmerzfrei und sicher koppelbar, also an- und entkoppelbar, mit dem extrakorporalen Blutkreislauf für insbesondere eine Blutbehandlung verbunden zu werden.

Die zwei Anschlüsse sind dafür vorgesehen und angepasst für das lösbare Koppeln aus der Haut des Patienten hervorzustehen und erlauben somit eine einfache und sichere Verbindung. Somit weist das vaskuläre Zugangsimplantat im Wesentlichen einen ersten Leitungs-/Strukturabschnitt auf, der im Körper implantiert ist, durch die Haut gegenüber der Umgebung abgegrenzt ist und eine Verbindung von der Arterie und der Vene mit dem ersten Leitungs-/Strukturabschnitt des Zugangsimplantats herstellt. Weiter weist das vaskuläre Zugangsimplantat einen zweiten Strukturabschnitt auf, der außerkörperlich durch die Haut hervorsteht und einen mechanischen / mechanisch ausgebildeten (Kopplungs-/Koppel-)Anschluss bildet, an dem der extrakorporale Blutkreislauf an- und abgekoppelt werden kann. Der erste Strukturabschnitt und der zweite Strukturabschnitt des vaskulären Zugangsimplantats sind (mechanisch und fluidtechnisch) miteinander verbunden. Somit wird eine definierte Schnittstelle für insbesondere eine Blutbehandlung bereitgestellt, welche schmerzfrei verbindbar ist, einen angenehmen Tragekomfort für den Patienten bietet sowie eine sichere und sterile Anbindung gewährleistet.

Erfindungsgemäß weist das vaskuläre Zugangsimplantat also einen arteriellen Anschluss auf, der dafür angepasst ist, aus einer Haut des Patienten hervorzustehen, und der mit der arteriellen Fluidleitung fest fluidverbunden ist und der dafür angepasst ist, als arterieller Zugang mit einem ersten (externen) Schlauch des extrakorporalen Blutkreislaufs ankoppelbar und entkoppelbar zu sein. Weiter weist das vaskuläre Zugangsimplantat einen venösen Anschluss auf, der dafür angepasst ist, aus einer Haut des Patienten hervorzustehen, und der mit der venösen Fluidleitung fest fluidverbunden ist und der dafür angepasst ist, als venöser Zugang mit einem zweiten (externen) Schlauch des extrakorporalen Blutkreislaufs verbunden zu werden. Das vaskuläre Zugangsimplantat ist insbesondere dafür ausgebildet, vollständig im Körper des Patienten aufgenommen zu werden, wobei nur der arterielle Anschluss/Anschlussstutzen und der venöse Anschluss/Anschlussstutzen von außen her einfach, sicher und schnell anschließbar sind und vorzugsweise durch eine Haut des Patienten aus dem Körper nach außen hervorstehen.

Konstruktiv wird der vorstehend prinzipielle Grundgedanke im Wesentlichen dadurch umgesetzt, dass das vaskuläre Zugangsimplantat den arteriellen Anschluss und den venösen Anschluss aufweist, der jeweils über eine Leitung an der arteriellen bzw. venösen Fluidleitung angeschlossen ist. Das andere Ende der Leitung (das der arteriellen bzw. venösen Fluidleitung abgewandte Ende der Leitung) weist jeweils einen separaten oder einen gemeinsamen Adapter bzw. eine Adapterstruktur zur Kopplung auf, der bzw. die derart ausgeformt ist, dass der Adapter durch die Haut durchsteht und von außen bzw. außerhalb des Körpers her gut erreichbar ist. In den Adapter kann dabei von außen beispielsweise mit einer Nadel in eine definierte Nadeleinstichstruktur des Adapters eingestochen werden, um über die Nadel eine Fluidverbindung herzustellen, oder der Adapter kann nadellos an- und entkoppelt werden, beispielsweise mittels eines Luer-Anschlusses.

Die Begriffe "koppelbar" bzw. "an- und entkoppelbar" bedeuten in diesem Zusammenhang, dass ein Ende eines (ersten) Fluidkanals mit einem anderen Ende eines unterschiedlichen (zweiten) Fluidkanals unmittelbar verbunden und gegeneinander fix positioniert und in dieser Position solange permanent gehalten werden, bis eine gewollte Entkopplung durch einen Anwender oder durch einen Steuer-Befehl gegeben wird, so dass eine sichere und lösbare Fluidverbindung zwischen beiden Fluidkanälen geschaffen wird.

Dabei weist der arterielle Anschluss ein arterielles Verbindungsschloss / eine arterielle Kupplung auf, welches mittels Formschluss und/oder Kraftschluss den ersten Schlauch fest anbindet und/oder der venöse Anschluss weist ein venöses Verbindungsschloss auf, welches mittels Formschluss und/oder Kraftschluss den zweiten Schlauch fest anbindet. Das Verbindungsschloss stellt jeweils sicher, dass die Leitung bzw. der Schlauch auch sicher, korrekt und fest mit bzw. an dem entsprechenden Anschluss fluidverbunden angeschlossen ist und auch verbleibt. Das Verbindungsschloss schließt sozusagen den Schlauch fest an den entsprechenden Anschluss an. Durch die formschlüssige und/oder kraftschlüssige Anbindung lässt sich der Schlauch auch wieder einfach und schnell, mittels Aufhebung des Formschlusses bzw. Kraftschlusses, von dem Anschluss lösen.

Ferner bindet das arterielle Verbindungsschloss (die arterielle Kupplung) und/oder das venöse Verbindungsschloss (die venöse Kupplung) über einen verstellbaren Hinterschnitt / eine verstellbare hinterschnittige Struktur, wie etwa einer Schlauchkupplung / einer Schnellkupplung oder einer Luer-Lock-Verbindung, den entsprechenden Schlauch formschlüssig und/oder über eine magnetische oder magnetisierbare Struktur, wie einer Magnetkupplung oder einer Hysteresekupplung, kraftschlüssig den jeweiligen Schlauch an. Die hinterschnittige und/oder magnetisierbare Struktur bietet den Vorteil einer lösbaren, schmerzfreien und sicheren Anbindung.

Vorteilhafte Ausführungsformen sind in den Unteransprüchen beansprucht werden nachfolgend erläutert.

Gemäß einem ggf. unabhängig beanspruchbaren Aspekt der Erfindung kann das vaskuläre Zugangsimplantat eine autonome Energieversorgung / eine autonome Energieversorgungseinrichtung aufweisen, die Energie für Komponenten des vaskulären Zugangsimplantats bereitstellt. Um autark zu agieren, benötigt das vaskuläre Zugangsimplantat elektrische Energie für seine Komponenten, insbesondere für seine elektromechanischen und elektrotechnischen Komponenten wie beispielsweise Sensoren. Diese Energie erhält es durch die autonome Energieversorgung.

Gemäß einer weiteren bevorzugten, ggf. unabhängig beanspruchbaren Ausführungsform ist es vorgesehen, dass die autonome Energieversorgung des vaskulären Zugangsimplantats einen Energie-Harvester / einen Nanogenerator aufweist, der Energie über eine Körpertemperatur des Patienten und/oder einer Bewegung des Patienten und/oder einer Pulsation eines Gefäßes oder der steuerbaren Arterie-Venen-Verbindung in elektrische Energie umwandelt. Die benötigte elektrische Energie kann das Zugangsimplantat mittels des Energie-Harvesters "gewinnen", der die Körpertemperatur oder die Bewegung oder die Pulsation als Energiequelle verwendet. Der Energie-Harvester bewirkt insbesondere mittels piezoleketrischem Effekt eine Umwandlung einer mechanischen Energie in eine elektrische Energie und/oder mittels thermoelektrischen Generatoren oder pyroelektrischen Kristallen aus Temperaturunterschieden mittels thermoelektrischen Effekts in elektrische Energie.

Insbesondere kann die autonome Energieversorgung einen Energiespeicher aufweisen, insbesondere einen Akkumulator wie einen Lithium-Ionen Akku, um elektrische Energie in der autonomen Energieversorgung des vaskulären Zugangsimplantats zu speichern. Mittels des Akkumulators kann sichergestellt werden, dass das Zugangsimplantat seinen Komponenten die benötigte elektrische Energie über einen Zeitraum zur Verfügung stellt, um autark zu agieren und seine Funktion zuverlässig zu erfüllen.

Gemäß einer weiteren Ausführungsform kann elektrische Energie der autonomen Energieversorgung mittels drahtloser/kontaktloser Energieübertragung, insbesondere über induktive Energieübertragung, zuführbar sein. Vorzugsweise kann der Energiespeicher der autonomen Energieversorgung mittels kontaktloser Energieübertragung aufladbar sein. Die autonome Energieversorgung des Zugangsimplantats weist hierfür vorzugsweise eine Antenne einer (Kopplungs-) Spule auf, um mittels induktiver Kopplung mit einem magnetischen Fluss im Nahfeld geladen zu werden. So kann das Zugangsimplantat an einen passenden Sender mit Stromversorgung (mit einer Sendespule) mit wenigen Zentimetern Abstand gehalten werden, und der Sender versorgt das Zugangsimplantat kontaktlos mit Energie.

Es kann zweckdienlich sein, wenn der Ladezustand des Energiespeichers visuell angezeigt wird, insbesondere über ein subkutanes Leuchtmittel wie einer LED, oder mittels drahtloser Übertragung auslesbar ist. Das Zugangsimplantat weist vorzugsweise ein Leuchtmittel / eine Lampe auf, insbesondere eine LED oder ein LCD Display, welche direkt unter der Haut des Patienten platziert wird, so dass die Lichtdurchlässigkeit der Haut ausreichend ist, um durch die Haut hindurch Informationen, wie etwa einen Ladezustand, einen Koppelstatus oder einen Therapiefortschritt, anzuzeigen.

In einer bevorzugten ggf. unabhängig beanspruchbaren Ausführungsform können der arterielle Anschluss und/oder der venöse Anschluss und/oder die steuerbare Arterie-Venen-Verbindung eine, insbesondere selbstdesinfizierende, Desinfektionseinheit / Sterilisiereinheit als eine Komponente des vaskulären Zugangsimplantats aufweisen, welche biozidisch oder antibakteriell wirkt und Bakterien und Keime abtötet oder zumindest deren Wachstum hindert. Die Desinfektionseinheit, welche in dem vaskulären Zugangsimplantat integriert ist, dient der Sicherstellung der Sterilität des arteriellen Anschlusses und/oder des venösen Anschlusses und/oder der steuerbaren Arterie-Venen-Verbindung, so dass das Zugangsimplantat autonom seine Anforderungen an eine Sterilität erfüllen kann. Ein Infektionsrisiko sinkt.

Vorzugsweise ist die Desinfektionseinheit / Sterilisiereinheit dafür angepasst, (aktiv) ein UV-Licht oder ein Plasma auf den arteriellen Anschluss und/oder den venösen Anschluss und/oder die Arterie-Venen-Verbindung zu emittieren und/oder die Desinfektionseinheit kann eine (passive) antibakterielle Beschichtung aufweisen und/oder die Desinfektionseinheit kann ein mittels Licht aktivierbares selbstdesinfizierendes Material aufweisen. Ist die Desinfektionseinheit als UV-Licht emittierende UV-Lampe ausgestaltet, so kann sie beispielsweise vor einer Anbindung an den extrakorporalen Blutkreislauf mittels kurzwellige Strahlung Bakterien und Keime abtöten und desinfiziert so aktiv die arteriellen bzw. venösen Anschlüsse sowie die Arterie-Venen-Verbindung. Werden der arterielle und/oder der venöse Anschluss mit einer antibakteriellen Beschichtung ummantelt bzw. ausgestattet, die als Desinfektionseinheit wirken, so wird eine passive Desinfizierung für einen längeren Zeitraum, insbesondere dauerhaft, bereitgestellt. Alternativ oder zusätzlich kann die Desinfektionseinheit einen auffüllbaren Behälter innerhalb des Zugangsimplantats aufweisen, in welchem ein Desinfektionsmittel/Sterilisiermittel gespeichert ist, das dem arteriellen und/oder venösen Anschluss zugeführt werden kann.

In einer weiteren ggf. unabhängig beanspruchbaren Ausführungsform kann das vaskuläre Zugangsimplantat ein Identifikationselement und/oder einen Transponder / Sender, insbesondere einen RFID-Chip, als eine Komponente aufweisen, zum berührungslosen Identifizieren des Zugangsimplantats mittels eines geeigneten Lesegerätes. Durch die eindeutige Identifizierung kann sichergestellt werden, dass der intrakorporale Blutkreislauf auch an den richtigen extrakorporalen Blutkreislauf angeschlossen wird. Auch können Patientendaten berührungslos an die Blutbehandlungsmaschine gesendet werden, um eine Therapie der Blutbehandlung entsprechend (automatisch) einzustellen.

Das vaskuläre Zugangsimplantat kann ferner vorzugsweise eine ggf. unabhängig beanspruchbare Kommunikationseinheit als eine Komponente aufweisen, welche dafür angepasst ist, Daten berührungslos zu empfangen und zu senden. Durch die Kommunikationseinheit kann eine Daten-Verbindung beispielsweise zwischen der extrakorporalen Blutbehandlungsmaschine oder einem Computer eines Arztes zur Eingabe von aktualisierten Daten und dem Zugangsimplantat hergestellt werden. So können beispielsweise nach einer Therapie mit einer Dialysemaschine aktuelle Daten in dem Zugangsimplantat gespeichert werden und eine Historie in dem Zugangsimplantat selber abgespeichert werden. Bei einer nächsten Behandlung werden diese Werte dann wieder der Blutbehandlungsmaschine zur Verfügung gestellt, welche die Werte auswertet und entsprechend eine Behandlung anpasst bzw. einstellt.

Gemäß einem ggf. unabhängig beanspruchbaren Aspekt der Erfindung kann das vaskuläre Zugangsimplantat eine Anschluss-Dislokationserkennung / Anschluss-Dislokationserkennungseinrichtung als eine Komponente des Zugangsimplantats an dem arteriellen Anschluss und/oder an dem venösen Anschluss aufweisen, die dafür angepasst ist, insbesondere mittels eines Hall-Sensors und/oder eines Reed-Sensors / Reed Relais, zu detektieren, ob der arterielle Anschluss bzw. der venöse Anschluss korrekt angekoppelt ist bzw. ob an dem arteriellen Anschluss bzw. an dem venösen Anschluss der erste bzw. zweite Schlauch korrekt angebunden ist. Die Anschluss-Dislokationserkennung dient als Sicherheits- und Überwachungseinrichtung des Zugangsimplantats, die ein ungewolltes Lösen des Schlauchs oder einen Blutfluss ohne korrekte Anbindung detektieren und beispielsweise ein entsprechendes Steuer- bzw. Alarmsignal senden kann. Hierdurch wird eine Anbindung noch sicherer.

Insbesondere kann die steuerbare Arterie-Venen-Verbindung gemäß einem weiteren, ggf. unabhängig beanspruchbaren Aspekt der Erfindung einen Strömungsquerschnitt und/oder einen Fluss und/oder einen Fluidwiderstand/Flusswiderstand der Fluidverbindung zwischen der arteriellen Fluidleitung und der venösen Fluidleitung kontinuierlich, insbesondere mittels eines Proportionalventils, oder diskret einstellen. Vorzugsweise weist die steuerbare Arterie-Venen-Verbindung zusätzlich ein Rückschlagventil auf, das einen ungewollten Rückfluss von der venösen Fluidleitung zu der arteriellen Fluidleitung unterbindet. Die steuerbare Arterie-Venen-Verbindung bzw. das steuerbare Proportionalventil erlaubt eine genaue Einstellung des Druckes und des Flusses, um insbesondere die Blutbehandlung optimal zu unterstützen. Die Arterie-Venen-Verbindung des Zugangsimplantats kann dabei diskret angesteuert werden, um beispielsweise zwei Zustände, nämlich einen Offen-Zustand, in dem die Arterie-Venen-Verbindung komplett geöffnet und die Fluidverbindung hergestellt ist, und einen Geschlossen-Zustand in dem die Arterie-Venen-Verbindung komplett geschlossen ist, zu realisieren, oder sie kann auch mehrere diskrete Zustände annehmen, um auch Zwischenzustände zwischen dem Offen-Zustand und dem Geschlossen-Zustand einzunehmen. Alternativ dazu ist es möglich, dass die Arterie-Venen-Verbindung kontinuierlich einstellbar ist, beispielsweise mittels eines Proportionalventils, um auf hämodynamische Veränderungen reagieren zu können. Einen Blutfluss in der Arterie-Venen-Verbindung kann individuell angepasst werden und es kann insbesondere durch eine Drosselung einer Koagulation vorbeugen und gleichzeitig dennoch eine Herzentlastung herbeiführen. Insbesondere wird die steuerbare Arterie-Vene-Verbindung geschlossen, wenn der Patient nicht an den extrakorporalen Blutkreislauf angebunden ist. Durch die steuerbare Arterie-Venen-Verbindung werden Nebeneffekte bei einer Blutbehandlung, wie etwa einer Herzhypertrophie oder eines Steal-Syndroms, vermieden

Vorzugsweise und ggf. unabhängig beanspruchbar, kann das vaskuläre Zugangsimplantat einen Flusssensor und/oder einen Drucksensor aufweisen, der einen Fluss bzw. einen Druck in der steuerbaren Arterie-Venen-Verbindung und/oder an dem arteriellen Anschluss und/oder an dem venösen Anschluss misst. Der Fluss bzw. der Druck dient insbesondere der Steuerung der Arterie-Venen-Verbindung und wird dieser zu Grunde gelegt. Der Flusssensor misst sowohl eine Quantität als auch ein Vorzeichen (mögliche Rezirkulation) des Blutflusses und ist als Parameter für eine Behandlung wichtig. Der Drucksensor misst einen Druck, insbesondere der Arterie-Venen-Verbindung, und dient, insbesondere zusammen mit dem gemessenen Fluss, als weiterer Parameter für die Steuerung.

Gemäß einer weiteren bevorzugten und ggf. unabhängig beanspruchbaren Ausführungsform kann das vaskuläre Zugangsimplantat eine zentrale Steuereinheit aufweisen, mittels derer die Komponenten des vaskulären Zugangsimplantats gesteuert werden. Die zentrale Steuereinheit empfängt beispielsweise Daten von Komponenten wie etwa Sensoren, verarbeitet diese und sendet Daten an Komponenten, wie etwa die Kommunikationseinheit. Mittels der zentralen Steuereinheit kann das Zugangsimplantat automatisiert gesteuert werden.

Es ist von Vorteil, wenn zwischen der arteriellen Fluidleitung und dem arteriellen Anschluss ein steuerbares Ventil, insbesondere ein elektromechanisches Ventil, vorgesehen ist und/oder zwischen der venösen Fluidleitung und dem venösen Anschluss ein Rückschlagventil vorgesehen ist. Sollte die Verbindung an den extrakorporalen Blutkreislauf an dem arteriellen Anschluss unterbrochen werden, so kann das steuerbare Ventil schnell geschlossen werden und ein ungewollter Blutverlust in die Umgebung wird unterbunden. Andererseits wird durch das Rückschlagventil an dem venösen Anschluss ein Sicherheitselement vorgesehen, das einen Blutfluss aus dem venösen Anschluss heraus unterbindet. Da dem venösen Anschluss bei einer Blutbehandlung nur Blut zugeführt aber nicht abgeführt werden soll, reicht ein Rückschlagventil für einen Verschluss des venösen Anschlusses aus. Insbesondere wird das aktiv gesteuerte Ventil des arteriellen Anschlusses mittels der zentralen Steuereinheit gesteuert.

Es ist von Vorteil, wenn sowohl der arterielle Anschluss als auch der venöse Anschluss in einem einzigen vorzugsweise einteiligen Adapter, insbesondere in Form eines Doppel-Steckers, ausgebildet sind, der nur gemeinsam mit einem entsprechenden Gegenadapter koppelbar ist. Dadurch wird sichergestellt, dass nicht ein Anschluss, beispielsweise der arterielle Anschluss, mit einem Schlauch verbunden und der andere Anschluss, beispielsweise der venöse Anschluss von einem Schlauch getrennt ist. Durch den einzigen Adapter kann eine Kopplung an den arteriellen und den venösen Anschluss nur gleichzeitig erfolgen.

Gemäß einer bevorzugten und ggf. unabhängig beanspruchbaren Ausführungsform kann das vaskuläre Zugangsimplantat ein Gehäuse aufweisen, welches, bis auf die arterielle und venöse Fluidleitung sowie den arteriellen und venösen Anschluss, alle Komponenten des Zugangsimplantats, insbesondere die elektrischen Komponenten, komplett ummantelt / umschließt / umfasst, um eine Sterilbarriere zwischen den Komponenten und dem Körper zu schaffen, wobei das Gehäuse auf seiner Außenseite ein biokompatibles Material aufweist. Mittels des Gehäuses lassen sich verschiedene, auch nicht biokompatible, Komponenten in dem Zugangsimplantat integrieren, was insbesondere für elektrotechnische Bauteile von immenser Bedeutung ist. Alternativ kann das vaskuläre Zugangsimplantat eine (relativ) starre biokompatible Verbindungsstruktur aufweisen, welche die biokompatiblen Komponenten des vaskulären Zugangsimplantats starr miteinander verbindet.

Insbesondere weist der arterielle und/oder venöse Anschluss einen Dichtring auf. Dieser sichert eine verlustlose Blutverbindung.

Gemäß einem ggf. unabhängig beanspruchbaren Aspekt der Erfindung kann bei dem Zugangsimplantat ein Abstand zwischen der arteriellen Fluidleitung und der venösen Fluidleitung einstellbar sein. So wird gewährleistet, dass das Zugangsimplantat für verschiedene Anatomien einsetzbar ist, da der Abstand zwischen einer Arterie und einer Vene bei Patienten meist unterschiedlich ist. Der einstellbare Abstand kann beispielsweise durch eine in ihrer Länge einstellbare Arterie-Venen-Verbindung realisiert werden.

Daneben betrifft die Erfindung ein vaskuläres Zugangsimplantatsystem mit einem vaskulären Zugangsimplantat und mit einem extrakorporalen Blutkreislauf. Hierbei ist ein erfindungsgemäßes vaskuläres Zugangsimplantat in das vaskuläre Zugangsimplantatsystem eingesetzt, wobei an einem Schlauchende eines ersten Schlauchs des extrakorporalen Blutkreislaufs ein arterieller Gegenadapter vorgesehen ist, der mit einem arteriellen Adapter des arteriellen Anschlusses form- und/oder kraftschlüssig ankoppelbar ist, und/oder an einem Schlauchende eines zweiten Schlauchs des extrakorporalen Blutkreislaufs ein venöser Gegenadapter vorgesehen ist, der mit einem venöser Adapter des venösen Anschlusses form- und/oder kraftschlüssig ankoppelbar ist. Adapter und Gegenadapter bilden, ähnlich wie bei einer Stecker-Steckdosen-Verbindung / Steckverbindung, ein aufeinander abgestimmtes System des Zugangsimplantatsystems. Der Adapter passt auf den entsprechenden Gegenadapter, um eine Fluidverbindung zwischen dem Adapter und dem Gegenadapter herzustellen. Adapter und Gegenadapter können dabei unterschiedlich gestaltet sein. Beispielsweise können sie als komplementäre Schnellkupplungbauteile oder als Luer-Lock-Verbindung gestaltet sein. Das System des Adapters und des Gegenadapters des Zugangsimplantatsystems bildet eine definierte Schnittstelle und gewährleistet einen sicheren Anschluss des intrakorporalen Blutkreislaufs an den extrakorporalen Blutkreislauf.

### Kurzbeschreibung der Figuren

Die Erfindung wird nachfolgend anhand einer bevorzugten Ausführungsform mithilfe von begleitenden Figuren näher erläutert. Es zeigen:
- Fig. 1: eine schematische Draufsicht auf ein erfindungsgemäßes vaskuläres Zugangsimplantat eines erfindungsgemäßen Zugangsimplantatsystems gemäß einer bevorzugten Ausführungsform,
- Fig. 2: eine schematische Draufsicht auf das in einen Arm eines Patienten eingesetzte vaskuläre Zugangsimplantat aus Fig. 1, und
- Fig. 3: eine schematische Diagrammansicht des vaskulären Zugangsimplantats und Zugangsimplantatsystems aus Fig. 1.

### Detaillierte Beschreibung bevorzugter Ausführungsformen

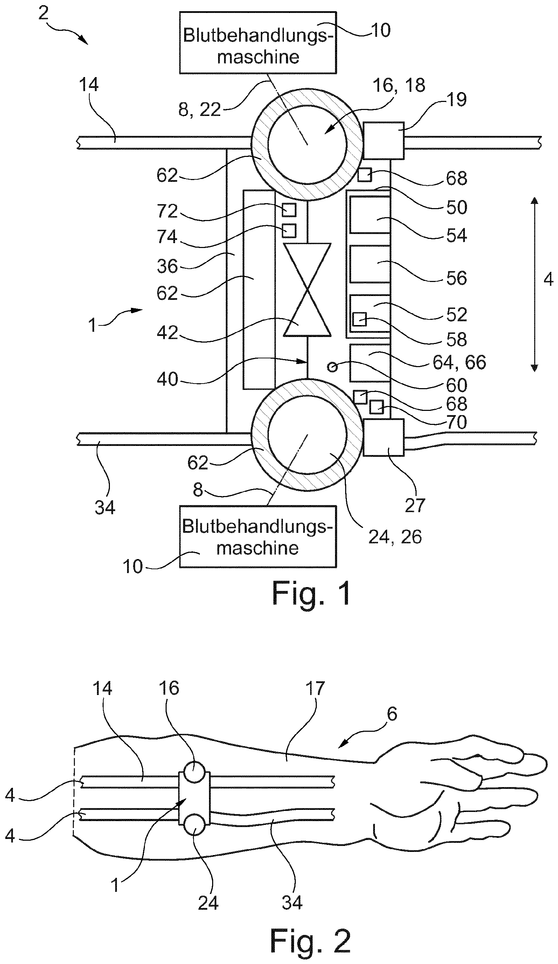

Figuren 1 bis 3 zeigen ein erfindungsgemäßes vaskuläres Zugangsimplantat 1 sowie ein erfindungsgemäßes Zugangsimplantatsystem 2 gemäß einer bevorzugten Ausführungsform mit einer Vielzahl an Komponenten.

Das Zugangsimplantat 1 dient als Schnittstelle zwischen einem intrakorporalen Blutkreislauf 4 eines Patienten 6 sowie einem extrakorporalen Blutkreislauf 8, um Blut dem Patienten 6 zu entnehmen, mittels einer Blutbehandlungsmaschine 10 in Form einer Dialysemaschine zu behandeln und anschließend dem Patienten 6 wieder zuzuführen. Mittels des extrakorporalen Blutkreislaufs 8 können unterschiedliche Behandlungen des Bluts durchgeführt werden, wie etwa eine Hämodialyse, eine Hämodiafiltration, eine Ultrafiltration oder eine (therapeutische) Apherese, um das Blut von Toxinen zu reinigen.

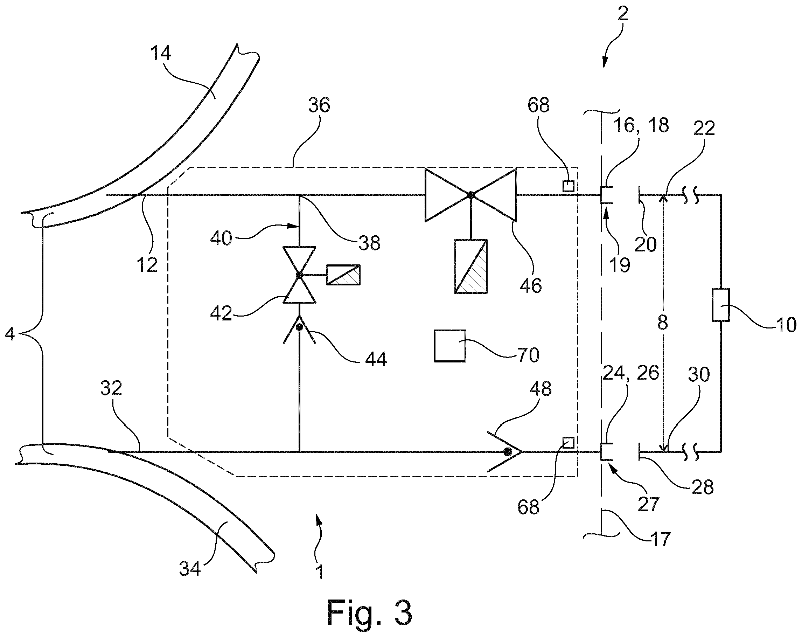

Das Zugangsimplantat 1 weist eine arterielle Fluidleitung 12 (siehe Fig. 3) beispielsweise in Form einer flexiblen arteriellen Silikonleitung oder einer Leitung aus einem geeigneten Gefäßersatzmaterial auf, die in eine Arterie 14 des Patienten 6 hineinsteht und mit dieser fluidverbunden ist. Alternativ kann die arterielle Fluidleitung 12 auch fest mit der Arterie 14 vernäht werden. Über die arterielle Fluidleitung 12 kann der Arterie 14 des Patienten 6 eine Blutmenge abgezogen bzw. entnommen/entzogen werden.

Die arterielle Fluidleitung 12 leitet das entzogene Blut an einen starr ausgebildeten arteriellen Anschluss 16 weiter. Dieser arterielle Anschluss 16 steht durch eine Haut 17 des Patienten 6 hindurch, durchbricht sozusagen die Hautbarriere, so dass der arterielle Anschluss 16 von außen (außerhalb der Körpers) her gut erreichbar und an den extrakorporalen Blutkreislauf 8 an- und entkoppelbar ist. Der arterielle Anschluss 16 weist hierfür einen arteriellen Adapter 18 auf bzw. ist als arterieller Adapter 18 gestaltet, der dafür angepasst ist, an einen arteriellen Gegenadapter 20 des extrakorporalen Blutkreislaufs 8 formschlüssig und/oder magnetisch (also kraftschlüssig) ankoppelbar bzw. an- und entkoppelbar zu sein (siehe Fig. 3). Der arterielle Adapter 18 ist mit der arteriellen Fluidleitung 12 fluiddicht verbunden und bildet sozusagen den endständigen (Blut-)Auslass des Zugangsimplantats 1 an den extrakorporalen Blutkreislauf 8.

Der arterielle Adapter 18 des intrakorporalen Blutkreislaufs 4 und der arterielle Gegenadapter 20 des extrakorporalen Blutkreislaufs 8 bilden zusammen ein aufeinander abgestimmtes System des Zugangsimplantatsystems 2 und eine definierte Schnittstelle aus. Der arterielle Adapter 18 passt auf den entsprechenden arteriellen Gegenadapter 20, um eine verlustlose, einfache und lösbare Fluidverbindung zwischen dem Adapter 18 und dem Gegenadapter 20 herzustellen. Der Gegenadapter 20 bildet dabei einen endständigen Abschnitt eines ersten (arteriellen) Schlauchs 22 des extrakorporalen Blutkreislaufs 8 aus. Der Adapter 18 als ein Ende des (ersten) Fluidkanals (des intrakorporalen Blutkreislaufs 4) wird so mit dem Gegenadapter 20 als anderes Ende des (zweiten) Fluidkanals (des extrakorporalen Blutkreislaufs 8) unmittelbar verbunden und gegeneinander fix positioniert und in dieser Position solange permanent gehalten werden, bis eine gewollte Entkopplung durch einen Anwender gegeben wird. So ist eine sichere und lösbare Fluidverbindung zwischen beiden Blutkreisläufen 4, 8 geschaffen. Der arterielle Adapter 18 fungiert als arterielles Verbindungsschloss 19. Eine magnetische Maximalkraft einer Anziehung ist in der Regel ausreichend, um kraftschlüssig ein arterielles Verbindungsschloss 19 zu realisieren. Insbesondere ist das arterielle Verbindungsschloss 19 so ausgelegt, dass bei einem kräftigen Zug am Gegenadapter 20 und damit am Adapter 18, gleich in welche Richtung, sich die Verbindung löst und damit ein Risiko eines Herausreißens des Zugangsimplantats 1 mit größter Schadensfolge minimiert wird.

Analog zu der arteriellen Seite des Zugangsimplantats 1, weist das vaskuläre Zugangsimplantat 1 auch auf seiner venösen Seite einen starren venösen Anschluss 24 mit analogen Merkmalen eines venösen Adapters 26 auf, der mit einem entsprechenden venösen Gegenadapter 28 eines zweiten Schlauchs 30 des extrakorporalen Blutkreislaufs 8 kraft- und/oder formschlüssig verbunden werden kann. Der venöse Adapter 26 bildet somit ein venöses Verbindungsschloss 27 aus. Über die Verbindung mit dem venösen Anschluss 24 kann das gereinigte bzw. behandelte Blut wieder dem Zugangsimplantat 1 zugeführt werden. Das Blut fließt an eine mit dem venösen Anschluss 10 verbundene eine venöse Fluidleitung 32 in Form einer flexiblen venösen Silikonleitung, die in eine Vene 34 des Patienten 6 hineinsteht und mit dieser fluidverbunden ist. Somit ist eine Fluidverbindung zwischen dem zweiten Schlauch 30 und der Vene 34 des Patienten 6 hergestellt. Durch den arteriellen Anschluss 16 sowie dem venösen Anschluss 24 wird ein Fluidkreislauf hergestellt, der den intrakorporalen Blutkreislauf 4 des Patienten 6 mit dem extrakorporalen Blutkreislauf 4 verbindet.

Die Funktionsweise des vaskulären Zugangsimplantats 1 gemäß der bevorzugten Ausführungsform wird nachfolgend erläutert. Anstatt also, wie beispielsweise bei einem üblicherweise gebräuchlichen Shunt, bei jeder Behandlung des Patienten 6 mittels einer Stichs einer Kanüle durch die Haut 17 des Patienten 6 in das Gefäß (Arterie/Vene) des Patienten 6 einzustechen und so eine Blutentnahmestelle zu schaffen, um für eine extrakorporale Blutbehandlung das Blut abzuführen, und ebenfalls einen zweiten Einstich durchzuführen, um das gereinigte Blut wieder der Vene des Patienten 6 zuzuführen, wird mit dem Zugangsimplantat 1 eine vollständig implantierbare Schnittstellenvorrichtung zur Verfügung gestellt, welche permanent mit sowohl der Arterie 14 als auch der Vene des Patienten 34 verbunden ist und welche einen definierten gemeinsamen Anschluss mit zwei separaten Fluidverbindungen oder zwei separaten Anschlüssen (mit arteriellen Anschluss 16 und venösen Anschluss 24) an den extrakorporalen Blutkreislauf 8 bereitstellt. Der extrakorporale Blutkreislauf 8 kann mit dem Zugangsimplantat 1 (lösbar) und schmerzfrei gekoppelt werden, ohne dass Restriktionen wie etwa eine Anzahl an An- und Abkopplungen oder einer Ortsveränderlichkeit, wie dies bei Einstichen von Kanülen der Fall ist (Gefäßschädigung), eingehalten werden müssen.

Das Zugangsimplantat 1 weist ferner ein Gehäuse 36 auf, dessen Außenseite mit biokompatiblen Materialen gestaltet ist und durch seine strukturelle Ausgestaltung vollständig im Körper des Patienten 6 dauerhaft implantierbar ist. Lediglich der arterielle Anschluss 16 bzw. der arterielle Adapter 18 und der venöse Anschluss 24 bzw. der venöse Adapter 26 stehen aus der Haut 15 des Patienten hervor. Das Gehäuse 36 ummantelt, bis auf die hervorstehende arterielle Fluidleitung 12, den vorstehenden arteriellen Anschluss 16, die hervorstehende venöse Fluidleitung 32 und den vorstehenden venösen Anschluss 24, das Zugangsimplantat 1.

Zudem weist das vaskuläre Zugangsimplantat 1 von der arteriellen Fluidleitung 12 ausgehend eine Abzweigung 38 auf, die mit der venösen Fluidleitung 32 über eine steuerbare Arterie-Venen-Verbindung 40 verbunden ist, um die Arterie 14 mit der Vene 34, ähnlich eines Bypass, kurzzuschließen. In der Arterie-Venen-Verbindung 40 ist ein steuerbares elektromechanisches Proportionalventil 42 zwischengeschalten, um den Fluss in der Arterie-Venen-Verbindung 40 kontinuierlich zu steuern. Das Proportionalventil 42 kann ausgehend von einer Offen-Stellung kontinuierlich geschlossen und hiernach auch wieder geöffnet werden, wodurch auch Zustände zwischen einer Offen-Stellung und einer Geschlossen-Stellung realisiert werden. Über das Proportionalventil 42 kann innerhalb von Millisekunden ein Widerstand bzw. ein Fluss in der Arterie-Venen-Verbindung 40 geändert werden, um auf hämodynamische Veränderungen reagieren zu können. Einen Blutfluss in der Arterie-Venen-Verbindung 40 kann so individuell angepasst werden und es kann durch eine Drosselung einer Koagulation vorgebeugt und gleichzeitig eine Herzentlastung herbeigeführt werden. Ist der Patient 6 mittels des extrakorporalen Blutkreislaufs 8 an die Blutbehandlungsmaschine 10 angeschlossen, so wird die steuerbare Arterie-Vene-Verbindung 40 geöffnet und wenn der Patient 6 nicht mehr an den extrakorporalen Blutkreislauf 8 angebunden ist, geschlossen.

Um eine Rezirkulation des Bluts in die Arterie 14 in der Arterie-Venen-Verbindung 40 zu verhindern, ist in der Arterie-Venen-Verbindung 40 zudem ein Rückschlagventil 44 vorgesehen, welches nur die Flussrichtung von der Arterie 14 zu der Vene 34 hin zulässt.

Vor dem arteriellen Anschluss 8 bzw. stromaufwärts ist in der arteriellen Fluidleitung 12 ein steuerbares elektromechanisches Ventil 46 vorgesehen, welches zwischen einem Offen-Zustand und einem-Geschlossen Zustand schaltet. Alternativ kann das steuerbare Ventil 46 auch kontinuierlich einen Fluss ändern. Das steuerbare Ventil 46 ist dafür vorgesehen, dass bei einem Abkoppeln des arteriellen Gegenadapters 20 von dem arteriellen Anschluss 16 der Blutstrom sofort unterbrochen wird, um nicht unkontrolliert in die Umgebung abgegeben zu werden.

Ebenfalls ist vor dem venösen Anschluss 24 ein Rückschlagventil 48 in der venösen Fluidleitung 32 vorgesehen. Dieses muss nicht zwangsläufig aktiv betätigbar sein, da es druckabhängig den venösen Anschluss 24 bei Abkopplung gegenüber der Umgebung abschließt. Der venöse Anschluss 24 lässt mittels des Rückschlagventils 48 lediglich Blut in das Zugangsimplantat 1 einströmen. So verschließt das Rückschlagventil 48 den venösen Anschluss 24 sicher und gewährleistet, dass keine ungewollte Austrittsstelle des Blutsystems des Patienten 6 entsteht.

Um autonom und unabhängig agieren zu können bzw. aktuiert werden zu können, weist das Zugangsimplantat 1 eine autonome Energieversorgung 50 auf, die elektrische Energie für die (elektrotechnischen) Komponenten des Zugangsimplantats 1 bereitstellt. Zum einen weist die autonome Energieversorgung 50 einen Energiespeicher 52 in Form eines wieder aufladbaren Lithium-Ionen-Akkus auf, um elektrische Energie über einen längeren Zeitraum zu speichern und zum anderen weist die autonome Energieversorgung 50 einen ersten Energie-Harvester 54 in Form eines thermoelektrischen Generators auf, der Temperaturunterschiede mittels thermoelektrischen Effekts in elektrische Energie umwandelt und dem Energiespeicher 52 einspeist und einen zweiten Energie-Harvester 56 auf, der Bewegungsenergie des Patienten 6 in elektrische Energie umwandeln kann und dem Energiespeicher 52 zuführt. Mit den beiden Energie-Harvestern 54, 56 kann die autonome Energieversorgung 50 des Zugangsimplantats 1, ohne eine Notwendigkeit an eine externe Aufladestation angekoppelt werden zu müssen, kontinuierlich mit elektrischer Energie gespeist werden. Um dem Energiespeicher 52 der autonomen Energieversorgung 50 gegebenenfalls auch unabhängig zu den Energie-Harvestern 54, 56 aufladen zu können, weist die autonome Energieversorgung 50 eine Antenne einer Kopplungsspule bzw. Ladespule 58 auf, um mittels induktiver Energieübertragung im Nahfeld bei Bedarf auch kabellos geladen werden zu können. Damit kann das Zugangsimplantat 1 an eine komplementäre externe Koppelspule in einigen Zentimetern Entfernung gehalten werden, um den Energiespeicher 52 über die Ladespule 58 induktiv berührungslos zu laden.

Das Zugangsimplantat 1 weist zudem einen Indikator 60 in Form von einer LED auf, um den Ladezustand des Energiespeichers 52 anzuzeigen. Die LED hat eine ausreichende Leuchtkraft, um durch die Haut 17 des Patienten 6 hindurch visuell wahrgenommen zu werden. So kann beispielsweise der Ladezustand des Energiespeichers 52 bei niedrigem Energiestand in Form von der rot blinkenden LED angezeigt werden.

Um den arteriellen Anschluss 16 sowie den venösen Anschluss 24 für eine Ankopplung / Verbindung zu sterilisieren, weist das Zugangsimplantat 1 eine antibakteriell wirkende Desinfektionseinheit 62 in Form einer UV-Lampe (Emitter) als eine Komponente des Zugangsimplantats 1 auf. Die Desinfektionseinheit 62 kann mittels Emission von UV-Licht Oberflächen der beiden Anschlüsse 16, 24 desinfizieren bzw. sterilisieren. Zusätzlich dazu weist der arterielle und venöse Anschluss 16, 24 ein Material mit antibakterieller Wirkung bzw. eine antibakterielle Beschichtung auf, um eine lang anhaltende passive Desinfizierung der Anschlüsse 16, 24 zu erzielen. Die Arterie-Venen-Verbindung 40 kann ebenfalls mittels UV-Licht der Desinfektionseinheit 62 bestrahlt und desinfiziert werden.

Daten können mittels einer Kommunikationseinheit 64 als eine weitere Komponente, insbesondere mittels einer Bluetooth^{®} oder WLAN Verbindung, an die extrakorporale Blutbehandlungsmaschine 10 übertragen werden, um eine Therapie optimal einzustellen. Auch können über die Kommunikationseinheit 64 Daten an das Zugangsimplantat übertragen werden. Die Kommunikationseinheit 64 ist also dafür ausgelegt, drahtlos mit anderen Maschinen bzw. Systemen zu kommunizieren.

Mit der Kommunikationseinheit 64 ist zudem ein innerhalb des Gehäuses 36 des vaskulären Zugangsimplantats 1 angeordnetes Identifikationselement 66 (als weitere Komponente) in Form eines RFID-Chips oder einer NFC-Spule verbunden. Mittels des Identifikationselements 66 kann der Patient 6 eindeutig von anderen Maschinen identifiziert werden. Beispielsweise kann ein externes Lesegerät der Blutbehandlungsmaschine 10, neben den über die Kommunikationseinheit 64 übermittelten Daten, drahtlos die Daten des Identifikationselements 66 ausgelesen und eine Therapie entsprechend an den Patienten 3 anpassen und einstellen. Da beispielsweise eine NFC-Spule nur eine sehr geringe Reichweite hat, kann eine Daten-Sicherheit erhöht werden, da nur Daten im Nahfeld übertragen werden. Ein Auslesen der Patientendaten durch eine unerlaubte Person wird erschwert, da die Daten in direkter Umgebung abgegriffen werden müssten. Insbesondere können die Daten in dem Zugangsimplantat 1 auch verschlüsselt werden, wobei vorzugsweise die Kommunikationseinheit 64 oder die zentrale Steuereinheit 70 die Verschlüsselung durchführt. Die Blutbehandlungsmaschine 10, die den Schlüssel der Verschlüsselung kennt, kann diese verschlüsselt übertragenen Daten dann entsprechend entschlüsseln. Analog kann das Zugangsimplantat 1 auch verschlüsselte Daten von der Blutbehandlungsmaschine 10 entschlüsseln.

Um einen korrekten Anbindungszustand an dem arteriellen und venösen Anschluss 16, 24 feststellen zu können, weist das Zugangsimplantat 1 eine Anschluss-Dislokationserkennung 68 (als weitere Komponente) auf, die mittels Reed-Sensors und/oder Hall-Sensors erkennen kann, ob der jeweilige Gegenadapter 20, 28 korrekt an dem Adapter 18, 26 angeschlossen ist. Die Anschluss-Dislokationserkennung 68 misst einen magnetischen bzw. sich ändernden magnetischen Fluss und bestimmt darüber, ob eine korrekte Ankopplung an dem jeweiligen Anschluss 16, 24 vorliegt. Eine korrekte Anbindung ist von enormer Bedeutung, da sie als Sicherheitseinrichtung fungiert und ein gewolltes oder ungewolltes Lösen des Schlauchs 22, 30 detektiert und hiernach mittels des steuerbaren Ventils 46 eine Fluidverbindung unterbricht, um einen Blutverlust zu vermeiden. Insbesondere kann das Rückschlagventil 48 auch (elektromechanisch) verschließbar sein. Löst sich beispielsweise ungewollt nur der arterielle Schlauch 22, dann kann das Rückschlagventil 48 geschlossen werden, wodurch an der Blutbehandlungsmaschine 10 ein Druckalarm ausgelöst wird und eine Blutpumpe gestoppt wird. Alternativ kann das Zugangsimplantat 1 auch beide Ventile 46, 48 schließen und mittels der Kommunikationseinheit 64 einen Steuer-Befehl bzw. Alarm-Befehl an die Blutbehandlungsmaschine 10 senden, die dann die Blutbehandlung stoppt und ein zu dem Fehler zugehöriges Alarmsignal bzw. Fehler-Informationen am Display anzeigt.

Das vaskuläre Zugangsimplantat 1 weist ferner eine zentrale Steuereinheit 70 auf, welche empfangene Daten/Signale verarbeitet und alle (elektrotechnischen) Komponenten des Zugangsimplantats 1 ansteuern kann. Die Steuereinheit 70 ist somit der zentrale Knotenpunkt des Zugangsimplantats 1 für alle elektrotechnischen Komponenten. Die zentrale Steuereinheit 70 steuert das Proportionalventil 42 und empfängt Daten von diesem hinsichtlich eines aktuellen (Ventil-)Zustands, steuert das steuerbare Ventil 46 und empfängt auch von diesem Daten zu seinem Zustand, steuert die Autonome Energieversorgung 50 mit dem Energiespeicher 52, den beiden Energie-Harvestern 54, 56 und der Ladespule 58 und erhält Daten von diesem zu beispielsweise einem aktuellen Ladezustand, einer aktuellen Maximalladekapazität, einer Energie-Gewinnung sowie einem Energie-Verbrauch durch die Komponenten, steuert den Indikator 60 und optional einen akustischen Signalgeber, steuert die Desinfektionseinheit 62 und empfängt ggf. Fehlermeldungen, falls beispielsweise die UV-Lampe defekt ist, steuert die Kommunikationseinheit 64 und empfängt von dieser Daten, steuert ggf. das Identifikationselement 66 und empfängt Daten von der Anschluss-Dislokationserkennung 68. Zudem speichert die Steuereinheit 70 relevante Daten, wie etwa Patientendaten, Therapiedaten, Komponentendaten, Referenzdaten in ihrem Speicher.

Die Arterie-Venen-Verbindung 40 weist einen Drucksensor 72 und einen Flusssensor 74 auf, der den Druck sowie den Fluss in der Arterie-Venen-Verbindung 40 misst und der zentralen Steuereinheit 70 als Daten zur Verfügung stellt. Die Daten bzw. Parameter des Drucksensors 72 und des Flusssensors 74 stellen die Basis für die Steuerung des Proportionalventils 42 der steuerbaren Arterie-Venen-Verbindung 40, mithilfe derer Nebeneffekte einer Blutbehandlung, wie einer Herzhypertrophie, vermieden werden können.

Mit dem erfindungsgemäßen Zugangsimplantat 1 bzw. dem erfindungsgemäßen Zugangsimplantatsystem wird also dem Patienten eine Vorrichtung bzw. ein System zur schmerzfreien Kopplung mit erhöhter Sicherheit, vermindertem Infektionsrisiko, einer verbesserten Durchgängigkeit und besseren Therapiebedingungen mit geringerer Vasodilatation, geringerem Risiko einer Herzerkrankung und geringerer Anfälligkeit eines ungewollten Blutverlustes zur Verfügung gestellt.

### Bezugszeichenliste

- 1: Vaskuläres Zugangsimplantat
- 2: Vaskuläres Zugangsimplantatsystem
- 4: Intrakorporaler Blutkreislauf
- 6: Patient
- 8: Extrakorporaler Blutkreislauf
- 10: Blutbehandlungsmaschine
- 12: Arterielle Fluidleitung
- 14: Arterie Patient
- 16: Arterieller Anschluss
- 17: Haut Patient
- 18: Arterieller Adapter
- 19: Arterielles Verbindungsschloss
- 20: Arterieller Gegenadapter
- 22: Erster Schlauch
- 24: Venöser Anschluss
- 26: Venöser Adapter
- 27: Venöses Verbindungsschloss
- 28: Venöser Gegenadapter
- 30: Zweiter Schlauch
- 32: Venöse Fluidleitung
- 34: Vene Patient
- 36: Gehäuse
- 38: Abzweigung
- 40: Arterie-Venen-Verbindung
- 42: Proportionalventil
- 44: Rückschlagventil
- 46: Steuerbares Ventil
- 48: Rückschlagventil
- 50: Autonome Energieversorgung
- 52: Energiespeicher
- 54: Erster Energie-Harvester
- 56: Zweiter Energie-Harvester
- 58: Ladespule
- 60: Indikator
- 62: Desinfektionseinheit
- 64: Kommunikationseinheit
- 66: Identifikationselement
- 68: Anschluss-Dislokationserkennung
- 70: Zentrale Steuereinheit
- 72: Drucksensor
- 74: Flusssensor

## Patentansprüche

1. Vaskuläres Zugangsimplantat (1) für einen Anschluss eines intrakorporalen Blutkreislaufs (4) mit einem extrakorporalen Blutkreislauf (8), insbesondere zur Fluid-Verbindung mit einer extrakorporalen Blutbehandlungsmaschine (10),
mit einer arteriellen Fluidleitung (12), die dafür angepasst ist mit einer Arterie (14) eines Patienten (6) permanent verbunden zu sein,
mit einer venösen Fluidleitung (32), die dafür angepasst ist, mit einer Vene (34) des Patienten (6) permanent verbunden zu sein,
mit einer steuerbaren Arterie-Venen-Verbindung (40) zwischen der arteriellen Fluidleitung (12) und der venösen Fluidleitung (32), welche, insbesondere mittels eines steuerbaren Ventils (42), einen Fluidstrom zwischen der arteriellen Fluidleitung (12) und der venösen Fluidleitung (32) steuert,
**gekennzeichnet durch**
einen arteriellen Anschluss (16), der dafür angepasst ist, aus einer Haut des Patienten hervorzustehen, und der mit der arteriellen Fluidleitung (12) fest fluidverbunden ist und der dafür angepasst ist, als arterieller Zugang mit einem ersten Schlauch (22) des extrakorporalen Blutkreislaufs (8) ankoppelbar und entkoppelbar zu sein, und
einen venösen Anschluss (24), der dafür angepasst ist, aus einer Haut des Patienten hervorzustehen, und der mit der venösen Fluidleitung (32) fest fluidverbunden ist und der dafür angepasst ist, als venöser Zugang mit einem zweiten Schlauch (30) des extrakorporalen Blutkreislaufs (8) ankoppelbar und entkoppelbar zu sein,
wobei der arterielle Anschluss (16) ein arterielles Verbindungsschloss (19) aufweist, welches über eine hinterschnittige Struktur formschlüssig oder über eine magnetische oder magnetisierbare Struktur kraftschlüssig den ersten Schlauch (22) fest anbindet und/oder der venöse Anschluss (24) ein venöses Verbindungsschloss (27) aufweist, welches über eine hinterschnittige Struktur formschlüssig oder über eine magnetische oder magnetisierbare Struktur kraftschlüssig den zweiten Schlauch (30) fest anbindet.

2. Vaskuläres Zugangsimplantat (1) nach Anspruch 1 **dadurch gekennzeichnet, dass** das vaskuläre Zugangsimplantat (1) eine autonome Energieversorgung (50) aufweist, die Energie für Komponenten (42; 46; 62; 64; 66; 68; 70; 72; 74) des vaskulären Zugangsimplantats (1) bereitstellt.

3. Vaskuläres Zugangsimplantat (1) nach Anspruch 2, **dadurch gekennzeichnet, dass** die autonome Energieversorgung (50) einen Energie-Harvester (54; 56) aufweist, der Energie einer Körpertemperatur des Patienten (6) und/oder einer Bewegung des Patienten (6) und/oder einer Pulsation eines Gefäßes oder der steuerbaren Arterie-Venen-Verbindung (40) in elektrische Energie umwandelt und der autonomen Energieversorgung (50) bereitstellt.

4. Vaskuläres Zugangsimplantat (1) nach Anspruch 2 oder 3, **dadurch gekennzeichnet, dass** die autonome Energieversorgung (50) einen Energiespeicher (52) aufweist, insbesondere einen Lithium-Ionen Akku, um elektrische Energie in der autonomen Energieversorgung (50) des vaskulären Zugangsimplantat (1) zu speichern.

5. Vaskuläres Zugangsimplantat (1) nach einem der Ansprüche 2 bis 4, **dadurch gekennzeichnet, dass** elektrische Energie der autonomen Energieversorgung (50) mittels drahtloser/kontaktloser Energieübertragung, insbesondere über induktive Energieübertragung, zuführbar ist.

6. Vaskuläres Zugangsimplantat (1) nach Anspruch 5, **dadurch gekennzeichnet, dass** die autonome Energieversorgung (50) für die drahtlose Energieübertragung eine Antenne einer Kopplungsspule (58) aufweist, mittels derer über induktive Kopplung mit einem magnetischen Fluss im Nahfeld Energie der autonomen Energieversorgung (50) kontaktlos zuführbar ist.

7. Vaskuläres Zugangsimplantat (1) nach einem der Ansprüche 1 bis 6, **dadurch gekennzeichnet, dass** der arterielle Anschluss (16) und/oder der venöse Anschluss (24) und/oder die steuerbare Arterie-Venen-Verbindung (40) eine Desinfektionseinheit (62) als eine Komponente des vaskulären Zugangsimplantats (1) aufweist, die antibakterielle Wirkung hat.

8. Vaskuläres Zugangsimplantat (1) nach Anspruch 7, **dadurch gekennzeichnet, dass** die Desinfektionseinheit (62) ein UV-Licht oder ein Plasma auf den arteriellen Anschluss (16) und/oder den venösen Anschluss (24) und/oder die steuerbare Arterie-Venen-Verbindung (40) emittiert und/oder dass die Desinfektionseinheit (62) eine antibakterielle Beschichtung oder ein antibakterielles Material aufweist und/oder ein mittels Licht aktivierbares selbst-desinfizierendes Material aufweist.

9. Vaskuläres Zugangsimplantat (1) nach einem der Ansprüche 1 bis 8, **dadurch gekennzeichnet, dass** das vaskuläre Zugangsimplantat (1) ein Identifikationselement (66), insbesondere einen RFID-Chip, als eine Komponente des vaskulären Zugangsimplantats (1) aufweist, zum berührungslosen Identifizieren mittels eines Lesegerätes und/oder eine Kommunikationseinheit (64) als eine Komponente des vaskulären Zugangsimplantats (1) aufweist, welche dafür angepasst ist, Daten berührungslos zu empfangen und zu senden.

10. Vaskuläres Zugangsimplantat (1) nach einem der Ansprüche 1 bis 9, **dadurch gekennzeichnet, dass** das vaskuläre Zugangsimplantat (1) eine Anschluss-Dislokationserkennung (68) als eine Komponente des vaskulären Zugangsimplantats (1) an dem arterielle Anschluss (16) und/oder an dem venösen Anschluss (24) aufweist, die dafür angepasst ist, insbesondere mittels eines Hall-Sensors und/oder eines Reed-Sensors, zu detektieren, ob der arterielle Anschluss (16) bzw. der venösen Anschluss (24) korrekt angekoppelt ist.

11. Vaskuläres Zugangsimplantat (1) nach einem der Ansprüche 2 bis 10, **dadurch gekennzeichnet, dass** die autonome Energieversorgung (50) ausreichend Energie der Desinfektionseinheit (62) und/oder dem Identifikationselement (66) und/oder der Kommunikationseinheit (64) und/oder der Anschluss-Dislokationserkennung (68) als Komponenten des vaskulären Zugangssystems (1) bereitstellt.

12. Vaskuläres Zugangsimplantatsystem (2) mit einem vaskulären Zugangsimplantat und mit einem extrakorporalen Blutkreislauf (8),
**dadurch gekennzeichnet, dass** ein vaskuläres Zugangsimplantat (1) nach einem der Ansprüche 1 bis 11 eingesetzt ist, und
dass an einem Schlauchende eines ersten Schlauchs (22) des extrakorporalen Blutkreislaufs (8) ein arterieller Gegenadapter (20) vorgesehen ist, der mit dem arteriellen Anschluss (16) als Adapter (18) form- und/oder kraftschlüssig ankoppelbar ist, und/oder
dass an einem Schlauchende des zweiten Schlauchs (30) des extrakorporalen Blutkreislaufs (8) ein venöser Gegenadapter (28) vorgesehen ist, der mit dem venösen Anschluss (24) als Adapter (26) form- und/oder kraftschlüssig ankoppelbar ist.

## Claims

1. A vascular access implant (1) for connecting an intracorporeal blood circulation (4) to an extracorporeal blood circulation (8), in particular for a fluidic connection to an extracorporeal blood treatment machine (10),
comprising an arterial fluid line (12) adapted to be permanently connected to an artery (14) of a patient (6),
comprising a venous fluid line (32) adapted to be permanently connected to a vein (34) of the patient (6),
comprising a controllable artery-vein-connection (40) between the arterial fluid line (12) and the venous fluid line (32), which controls, in particular by means of a controllable valve (42), a fluid flow between the arterial fluid line (12) and the venous fluid line (32),
**characterized by**
an arterial port (16) which is adapted to protrude from a patient's skin and which is in fixed fluid connection with the arterial fluid line (12) and is adapted, as an arterial access, to be able to be coupled to and decoupled from a first hose (22) of the extracorporeal blood circulation (8), and
a venous port (24) which is adapted to protrude from a patient's skin, and which is in fixed fluid connection with the venous fluid line (32) and is adapted, as a venous access, to be able to be coupled to and decoupled from a second hose (30) of the extracorporeal blood circulation (8),
wherein the arterial port (16) comprises an arterial connecting lock (19) which firmly connects to the first hose (22) via an undercut structure in a form-fitting manner or via a magnetic or magnetizable structure in a force-fitting manner, and/or the venous port (24) comprises a venous connecting lock (27) which firmly connects to the second hose (30) via an undercut structure in a form-fitting manner or via a magnetic or magnetizable structure in a force-fitting manner.

2. The vascular access implant (1) according to claim 1, **characterized in that** the vascular access implant (1) comprises an autonomous energy supply (50) which provides energy for components (42; 46; 62; 64; 66; 68; 70; 72; 74) of the vascular access implant (1).

3. The vascular access implant (1) according to claim 2, **characterized in that** the autonomous energy supply (50) comprises an energy harvester (54; 56) which converts energy of a body temperature of the patient (6) and/or of a movement of the patient (6) and/or of a pulsation of a vessel or the controllable artery-vein-connection (40) into electrical energy and supplies it to the autonomous energy supply (50).

4. The vascular access implant (1) according to claim 2 or 3, **characterized in that** the autonomous energy supply (50) comprises an energy storage device (52), in particular a lithium ion accumulator, for storing electrical energy in the autonomous energy supply (50) of the vascular access implant (1).

5. The vascular access implant (1) according to one of the claims 2 to 4, **characterized in that** electrical energy can be supplied to the autonomous energy supply (50) by means of wireless/contact-less energy transmission, in particular via inductive energy transmission.

6. The vascular access implant (1) according to claim 5, **characterized in that** the autonomous energy supply (50) comprises an antenna of a coupling coil (58) for wireless energy transmission, by means of which energy can be supplied to the autonomous energy supply (50) in contact-less manner via inductive coupling with a magnetic flow in the near field.

7. The vascular access implant (1) according to one of the claims 1 to 6, **characterized in that** the arterial port (16) and/or the venous port (24) and/or the controllable artery-vein-connection (40) comprise a disinfection unit (62) as a component of the vascular access implant (1), which has an anti-bacterial effect.

8. The vascular access implant (1) according to claim 7, **characterized in that** the disinfection unit (62) emits UV light or a plasma to the arterial port (16) and/or the venous port (24) and/or the controllable artery-vein-connection (40) and/or that the disinfection unit (62) comprises an anti-bacterial coating or an anti-bacterial material and/or comprises a self-disinfecting material which can be activated by means of light.

9. The vascular access implant (1) according to one of the claims 1 to 8, **characterized in that** the vascular access implant (1) comprises an identification element (66), in particular an RFID chip, as a component of the vascular access implant (1), for contact-less identification by means of a reader and/or a communication unit (64) as a component of the vascular access implant (1), which is adapted to receive and send data in a contact-less manner.

10. The vascular access implant (1) according to one of claims 1 to 9, **characterized in that** the vascular access implant (1) comprises a port dislocation detection (68) as a component of the vascular access implant (1) at the arterial port (16) and/or at the venous port (24), which is adapted to detect, in particular by means of a Hall sensor and/or Reed sensor, if the arterial port (16) and the venous port (24) are correctly coupled.

11. The vascular access implant (1) according to one of claims 2 to 10, **characterized in that** the autonomous energy supply (50) supplies a sufficient amount of energy to the disinfection unit (62) and/or the identification element (66) and/or the communication unit (64) and/or the port dislocation detection (68) as components of the vascular access system (1).

12. A vascular access implant system (2) comprising a vascular access implant and an extracorporeal blood circulation (8),
**characterized in that** a vascular access implant (1) according to one of the claims 1 to 11 is employed, and
an arterial mating adapter (20) is provided at one hose end of a first hose (22) of the extracorporeal blood circulation (8), wherein the arterial mating adapter is able to be coupled to the arterial port (16) as adapter (18) in a form-fitting and/or force-fitting manner, and/or
a venous mating adapter (28) is provided at one hose end of the second hose (30) of the extracorporeal blood circulation (8), wherein the venous mating adapter is able to be coupled to the venous port (24) as adapter (26) in a form- and/or force-fitting manner.

## Revendications

1. Implant d'accès vasculaire (1) pour un raccord d'un circuit sanguin intracorporel (4) à un circuit sanguin extracorporel (8), en particulier pour une connexion fluidique à une machine de traitement sanguin extracorporel (10),
avec un conduit de fluide artériel (12) qui est adapté pour être connecté en permanence à une artère (14) d'un patient (6),
avec un conduit de fluide veineux (32) qui est adapté pour être connecté en permanence à une veine (34) du patient (6),
avec une connexion artère-veine (40) pouvant être commandée entre le conduit de fluide artériel (12) et le conduit de fluide veineux (32), qui, en particulier au moyen d'une vanne (42) pouvant être commandée, commande un écoulement de fluide entre le conduit de fluide artériel (12) et le conduit de fluide veineux (32),
**caractérisé par**
un raccord artériel (16) qui est adapté pour faire saillie d'une peau du patient et qui est connecté de manière fluidique fixe au conduit de fluide artériel (12) et qui est adapté pour pouvoir être couplé et découplé en tant qu'accès artériel à un premier tuyau (22) du circuit sanguin extracorporel (8), et
un raccord veineux (24) qui est adapté pour faire saillie à partir d'une peau du patient et qui est connecté de manière fluidique fixe au conduit de fluide veineux (32) et qui est adapté pour pouvoir être couplé et découplé en tant qu'accès veineux à un second tuyau (30) du circuit sanguin extracorporel (8),
dans lequel le raccord artériel (16) présente un verrou de connexion artériel (19) qui attache fixement le premier tuyau (22) par une structure en contre-dépouille ou par une structure magnétique ou magnétisable et/ou le raccord veineux (24) présente un verrou de connexion veineux (27) qui attache fixement le second tuyau (30) par une structure en contre-dépouille ou par une structure magnétique ou magnétisable.

2. Implant d'accès vasculaire (1) selon la revendication 1, **caractérisé en ce que** l'implant d'accès vasculaire (1) présente une alimentation en énergie autonome (50) qui fournit de l'énergie à des composants (42 ; 46 ; 62 ; 64 ; 66 ; 68 ; 70 ; 72 ; 74) de l'implant d'accès vasculaire (1).

3. Implant d'accès vasculaire (1) selon la revendication 2, **caractérisé en ce que** l'alimentation en énergie autonome (50) présente un collecteur d'énergie (54 ; 56) qui convertit de l'énergie d'une température corporelle du patient (6) et/ou d'un mouvement du patient (6) et/ou d'une pulsation d'un vaisseau ou de la connexion artère-veine (40) pouvant être commandée en énergie électrique et la met à disposition de l'alimentation en énergie autonome (50).

4. Implant d'accès vasculaire (1) selon la revendication 2 ou 3, **caractérisé en ce que** l'alimentation en énergie autonome (50) présente un dispositif de stockage d'énergie (52), en particulier un accumulateur lithium-ion, pour stocker de l'énergie électrique dans l'alimentation en énergie autonome (50) de l'implant d'accès vasculaire (1).

5. Implant d'accès vasculaire (1) selon l'une quelconque des revendications 2 à 4, **caractérisé en ce que** de l'énergie électrique peut être amenée à l'alimentation en énergie autonome (50) au moyen d'une transmission d'énergie sans fil/sans contact, en particulier par transmission d'énergie inductive.

6. Implant d'accès vasculaire (1) selon la revendication 5, **caractérisé en ce que** l'alimentation en énergie autonome (50) pour la transmission d'énergie sans fil présente une antenne d'une bobine de couplage (58), au moyen de laquelle de l'énergie peut être amenée sans contact à l'alimentation en énergie autonome (50) par couplage inductif avec un flux magnétique dans le champ proche.

7. Implant d'accès vasculaire (1) selon l'une quelconque des revendications 1 à 6, **caractérisé en ce que** le raccord artériel (16) et/ou le raccord veineux (24) et/ou la connexion artère-veine (40) pouvant être commandée présente une unité de désinfection (62) en tant que composant de l'implant d'accès vasculaire (1) qui a un effet antibactérien.

8. Implant d'accès vasculaire (1) selon la revendication 7, **caractérisé en ce que** l'unité de désinfection (62) émet une lumière UV ou un plasma sur le raccord artériel (16) et/ou le raccord veineux (24) et/ou la connexion artère-veine (40) pouvant être commandée et/ou **en ce que** l'unité de désinfection (62) présente un revêtement antibactérien ou un matériau antibactérien et/ou présente un matériau autodésinfectant activable par de la lumière.

9. Implant d'accès vasculaire (1) selon l'une quelconque des revendications 1 à 8, **caractérisé en ce que** l'implant d'accès vasculaire (1) présente un élément d'identification (66), en particulier une puce RFID, en tant que composant de l'implant d'accès vasculaire (1), pour une identification sans contact au moyen d'un lecteur et/ou une unité de communication (64) en tant que composant de l'implant d'accès vasculaire (1) qui est adapté pour recevoir et envoyer des données sans contact.

10. Implant d'accès vasculaire (1) selon l'une quelconque des revendications 1 à 9, **caractérisé en ce que** l'implant d'accès vasculaire (1) présente une détection de dislocation de raccord (68) en tant que composant de l'implant d'accès vasculaire (1) au niveau du raccord artériel (16) et/ou du raccord veineux (24), composant qui est adapté pour détecter, en particulier au moyen d'un capteur Hall et/ou d'un capteur Reed, si le raccord artériel (16) ou le raccord veineux (24) est correctement couplé.

11. Implant d'accès vasculaire (1) selon l'une quelconque des revendications 2 à 10, **caractérisé en ce que** l'alimentation en énergie autonome (50) fournit suffisamment d'énergie à l'unité de désinfection (62) et/ou à l'élément d'identification (66) et/ou à l'unité de communication (64) et/ou à la détection de dislocation de raccord (68) en tant que composants du système d'accès vasculaire (1).

12. Système d'implant d'accès vasculaire (2) avec un implant d'accès vasculaire et un circuit sanguin extracorporel (8),
**caractérisé en ce qu'**un implant d'accès vasculaire (1) selon l'une quelconque des revendications 1 à 11 est utilisé, et
**en ce qu'**un contre-adaptateur artériel (20), qui peut être couplé par complémentarité de formes et/ou à force avec le raccord artériel (16) en tant qu'adaptateur (18) est prévu à une extrémité de tuyau d'un premier tuyau (22) du circuit sanguin extracorporel (8), et/ou
**en ce qu'**un contre-adaptateur veineux (28), qui peut être couplé par complémentarité de forme et/ou à force avec le raccord veineux (24) est prévu à une extrémité de tuyau du second tuyau (30) du circuit sanguin extracorporel (8) en tant qu'adaptateur (26).
